# EUROPEAN PATENT APPLICATION

(11) **EP 4 509 816 A1**
(43) Date of publication of application: **19.02.2025**
(21) Application number: 23784740.5
(22) Date of filing: 03.04.2023
(51) Int. Cl.: G01N 3/00, B23K 31/00, G01N 3/60, G01N 25/18

(54) **METHOD FOR PREDICTING DEFORMING OR RESIDUAL STRESS, AND PROGRAM**

(30) Priority: 05.04.2022 JP 2022063121
(71) Applicant: University Public Corporation Osaka, Osaka-shi, Osaka 545-0051 (JP)
(72) Inventor: SHIBAHARA, Masakazu, Sakai-shi, Osaka 599-8531 (JP); IKUSHIMA, Kazuki, Sakai-shi, Osaka 599-8531 (JP); MAEDA, Shintaro, Sakai-shi, Osaka 599-8531 (JP); KATO, Takuya, Sakai-shi, Osaka 599-8531 (JP); TEZEN, Towa, Sakai-shi, Osaka 599-8531 (JP); YASUDA, Shogo, Sakai-shi, Osaka 599-8531 (JP); LEE, Jiho, Sakai-shi, Osaka 599-8531 (JP)
(74) Representative: De Bortoli, Eros
(86) International application number: PCT/JP2023/013846
(87) International publication number: WO 2023/195454

(57) **Abstract**

The present invention provides a prediction method (modified thermal shrinkage method) that can quickly and accurately predict a deformation or a residual stress caused by returning temperature of an object subjected to heating to room temperature. The prediction method of the present invention is a method for predicting a deformation or a residual stress caused by returning temperature of an object subjected to heating to room temperature, including: a condition setting step of setting a first shrinkage zone and a second shrinkage zone in an analytical model of the object, and setting a first change in temperature of the first shrinkage zone and a second change in temperature of the second shrinkage zone; and an analysis step of performing an elastic analysis or an elastic-plastic analysis by giving a first shrinkage strain calculated from the first change in temperature to the first shrinkage zone and by giving a second shrinkage strain calculated from the second change in temperature to the second shrinkage zone.

## Description

### TECHNICAL FIELD

The present invention relates to a method for predicting a deformation or a residual stress, and a program.

### BACKGROUND ART

In recent years, FEM thermal elastic-plastic analysis has been used as a method for predicting deformation during the assembling of large welded structures with high accuracy. This method can predict temperature field, stress field, and displacement field that change from moment to moment, and as a result, can predict welding deformation with high accuracy. However, when the structure is large or complex, the number of elements and nodes for analyzing increases, which requires a long calculation time and makes the analysis extremely difficult. The thermal shrinkage method is thus developed (see, for example, Non-Patent Literature 1). The thermal shrinkage method is a method for setting a shrinkage zone with the mechanical melting temperature as a threshold, and for modeling and analyzing the thermal shrinkage during the cooling process of a welded structure, and makes it possible to predict angular distortion in a short time.

### CITATION LIST

### NON-PATENT LITERATURE

Non-Patent Literature 1: Pressure Engineering, 2020, Vol. 58, No. 2, pp. 93-100

### SUMMARY OF INVENTION

### TECHNICAL PROBLEM

However, a transverse shrinkage and a residual stress distribution calculated by analysis using the conventional thermal shrinkage method have a large deviation from a transverse shrinkage and a residual stress distribution calculated using thermal elastic-plastic analysis.

The present invention was devised in view of such circumstances, and provides a prediction method (modified thermal shrinkage method) that can quickly and accurately predict a deformation or a residual stress caused by returning temperature of an object subjected to heating to room temperature.

### SOLUTION TO PROBLEM

The prediction method (modified thermal shrinkage method) of the present invention is a method for predicting a deformation or a residual stress caused by returning temperature of an object subjected to heating to room temperature. The method includes: a condition setting step of setting a first shrinkage zone and a second shrinkage zone in an analytical model of the object, and setting a first change in temperature of the first shrinkage zone and a second change in temperature of the second shrinkage zone; and an analysis step of performing an elastic analysis or an elastic-plastic analysis by giving a first shrinkage strain calculated from the first change in temperature to the first shrinkage zone and by giving a second shrinkage strain calculated from the second change in temperature to the second shrinkage zone.

### ADVANTAGEOUS EFFECTS OF INVENTION

According to the prediction method of the present invention, it is possible to accurately predict in a short time the deformation or the residual stress caused by returning temperature of an object subjected to heating to room temperature.

### BRIEF DESCRIPTION OF THE DRAWINGS

FIG. 1 (a) is an explanatory diagram of a conventional thermal shrinkage method, and FIG. 1 (b) is an explanatory diagram of a prediction method (modified thermal shrinkage method) of the present invention.
FIG. 2 is a flowchart of a prediction method of one embodiment of the present invention.
FIG. 3 is an analytical model used to predict deformation and residual stress.
FIG. 4 is a cross-sectional view of a welded part of the analytical model shown in FIG. 3, and showing the formation sequence of 10 welding passes.
FIG. 5 is a graph showing material constants used in the analysis.
FIG. 6 (a) and FIG. 6 (b) are maximum temperature distributions, FIG. 6 (c) and FIG. 6 (d) are diagrams showing first and second shrinkage zones set in an analysis using the modified thermal shrinkage method of the present invention, and FIG. 6 (e) and FIG. 6 (f) are diagrams showing shrinkage zones set in an analysis using a conventional thermal shrinkage method.
FIG. 7 is a graph showing angular distortion history when welding passes were formed sequentially.
FIG. 8 is a graph showing transverse shrinkage history when welding passes were formed sequentially.
FIG. 9 is a contour diagram showing residual stress distribution calculated for an analytical model after forming the 10th welding pass.
FIG. 10 is a graph showing the residual stress in the X direction along the dotted line A-B shown in FIG. 9(a) to FIG. 9(c).

### DESCRIPTION OF EMBODIMENTS

The prediction method of the present invention (modified thermal shrinkage method) is a method for predicting a deformation or a residual stress caused by returning temperature of an object subjected to heating to room temperature. The prediction method of the present invention includes: a condition setting step of setting a first shrinkage zone and a second shrinkage zone in an analytical model of the object, and setting a first change in temperature of the first shrinkage zone and a second change in temperature of the second shrinkage zone; and an analysis step of performing an elastic analysis or an elastic-plastic analysis by giving a first shrinkage strain calculated from the first change in temperature to the first shrinkage zone and by giving a second shrinkage strain calculated from the second change in temperature to the second shrinkage zone.

The prediction method of the present invention preferably includes a step of calculating a maximum temperature distribution of the object, and the condition setting step is preferably a step of setting the first shrinkage zone, the second shrinkage zone, the first change in temperature, and the second change in temperature based on the maximum temperature distribution. This makes it possible to appropriately set the first shrinkage zone, the second shrinkage zone, the first change in temperature, and the second change in temperature.

The condition setting step is preferably a step of setting a zone where the maximum temperature is equal to or higher than a first temperature T₁ as a first shrinkage zone, and setting a zone where the maximum temperature is lower than the first temperature T₁ and higher than a second temperature T₂ as a second shrinkage zone.

In the condition setting step, it is preferable to set a third shrinkage zone in the analytical model and set a third change in temperature of the third shrinkage zone, and in the analysis step, it is preferable to perform the elastic analysis or the elastic-plastic analysis by giving a third shrinkage strain calculated from the third change in temperature to the third shrinkage zone. This makes it possible to improve the prediction accuracy of the prediction method of the present invention.

Preferably, in the condition setting step, a plurality of shrinkage zones is set in the analytical model, and a change in temperature of each shrinkage zone is set, and in the analysis step, the elastic analysis or the elastic-plastic analysis is performed by giving a shrinkage strain calculated from each change in temperature to the corresponding shrinkage zone. The plurality of shrinkage zones includes the first, second, and third shrinkage zones. The condition setting step is preferably a step of setting the plurality of shrinkage zones and the change in temperature of each shrinkage zone based on the maximum temperature distribution.

Preferably, the prediction method of the present invention is a method for predicting the deformation or the residual stress caused by returning temperature of the object subjected to multiple heating to room temperature, wherein the condition setting step is performed for each heating, and the analysis step is performed sequentially for each heating according to the heating order.

Preferably, the elastic analysis or the elastic-plastic analysis in the analysis step is performed with idealized explicit FEM.

The present invention also provides a program adapted to cause a computer to execute the prediction method of the present invention.

Below, one embodiment of the present invention will be described with reference to the drawings. The configurations shown in the drawings and the following description are examples, and the scope of the present invention is not limited to those shown in the drawings and the following description.

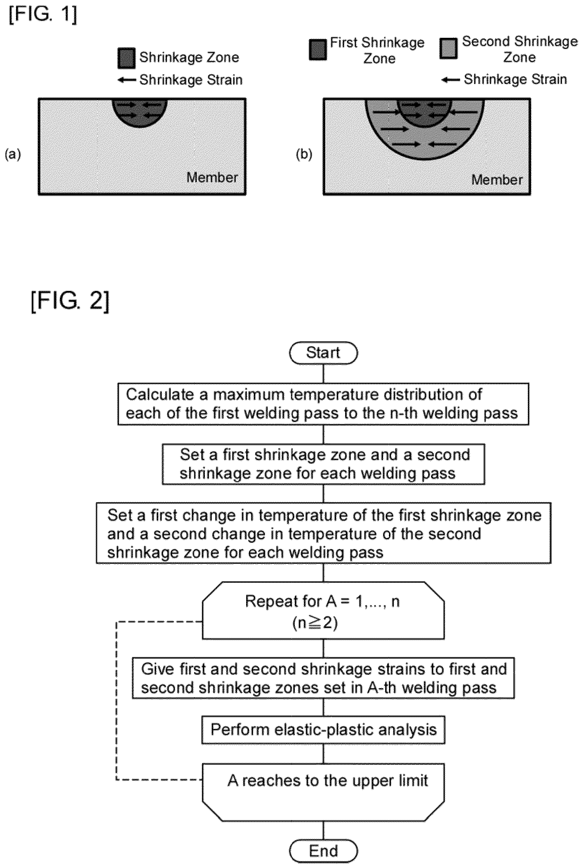

FIG. 1(a) is an explanatory diagram of a conventional thermal shrinkage method, and FIG. 1(b) is an explanatory diagram of a modified thermal shrinkage method of this embodiment.

In the conventional thermal shrinkage method, as shown in FIG. 1(a), only one shrinkage zone is set in the analytical model with the mechanical melting temperature as a threshold, and an elastic-plastic analysis is performed by uniformly giving a shrinkage strain to this shrinkage zone.

In contrast, in the modified thermal shrinkage method (prediction method) of this embodiment, a plurality of shrinkage zones (e.g., first and second shrinkage zones) are set in the analytical model as shown in FIG. 1(b), and an elastic analysis or an elastic-plastic analysis is performed by uniformly giving different shrinkage strain to each of these shrinkage zones. This makes it possible to predict deformation or residual stress with high accuracy. This was made clear by a simulation performed by the inventors of this application.

FIG. 2 is a flowchart of the prediction method of this embodiment, and in this flowchart, multi-layer welding is performed to form a first welding pass to an n-th welding pass.

The prediction method (modified thermal shrinkage method) of this embodiment is a method for predicting the deformation or the residual stress caused by returning temperature of an object subjected to heating to room temperature. The prediction method of this embodiment includes: a condition setting step of setting the first shrinkage zone and the second shrinkage zone in the analytical model of the object, and setting a first change in temperature of the first shrinkage zone and a second change in temperature of the second shrinkage zone; and an analysis step of performing the elastic analysis or the elastic-plastic analysis by giving a first shrinkage strain calculated from the first change in temperature to the first shrinkage zone and by giving a second shrinkage strain calculated from the second change in temperature to the second shrinkage zone.

The prediction method of this embodiment can include a step of calculating a maximum temperature distribution of the object.

A program of this embodiment is also provided to cause a computer to execute the prediction method of this embodiment.

The object is an object for the prediction, and is an object that undergoes a thermal cycle in which a locally heated zone returns to room temperature. This thermal cycle is, for example, a thermal cycle associated with bead-on welding, groove welding, fillet welding, seam welding, plug welding, slot welding, multi-layer welding, multi-pass welding, metal additive manufacturing (e.g., 3D printer, 3D metal additive manufacturing), strain relief by heating, cutting by heating (e.g., melt-cutting), bending by heating (e.g., linear heating), thermal spraying, etc. When the object is subjected to such a thermal cycle, shrinkage strain occurs during the cooling process, and deformation and residual stress occur in the object.

In the prediction method of this embodiment, first, the analytical model of the object (point cloud data representing the shape of the object) can be created. The analytical model is divided into multiple elements (meshes), and each vertex of each element becomes a node. The analytical model may be point cloud data representing the shape of any one of the following objects: butt joint, lap joint, double-sided strapped joint, single-sided strapped joint, corner j oint, T-joint, cruciform joint, edge joint, metal additive manufacturing, strain relief, and bending.

Then, a heat conduction analysis is performed using the created analytical model, data on the material of the object (specifically, specific heat, heat conduction coefficient, etc.), and heating conditions (specifically, heat input, heat source distribution parameters, torch speed, coordinates of the heating start point, coordinates of the heating end point, etc.) to calculate the maximum temperature distribution. When the object is heated multiple times (for example, multi-layer welding, multi-pass welding, metal additive manufacturing, etc.), the heat conduction analysis can be performed to calculate the maximum temperature distribution for each heating. In the flowchart of FIG. 2, the first to n-th welding passes are formed, so the maximum temperature distribution can be calculated for each welding pass. The maximum temperature distribution may also be derived from a theoretical formula, etc.

Next, the first shrinkage zone and the second shrinkage zone are set in the analytical model based on the maximum temperature distribution. When the object is heated multiple times (e.g., multi-layer welding, multi-pass welding, metal additive manufacturing, etc.), the first shrinkage zone and the second shrinkage zone can be set for each heating. In the flowchart of FIG. 2, the first welding pass to the n-th welding pass are formed, so the first shrinkage zone and the second shrinkage zone can be set for each welding pass.

For example, in the maximum temperature distribution, a zone where the maximum temperature is equal to or higher than a first temperature T₁ can be set as the first shrinkage zone, and a zone where the maximum temperature is lower than T₁ and higher than a second temperature T₂ can be set as the second shrinkage zone. The first temperature T₁ can be set as the mechanical melting temperature of the material of the object, for example. In addition, a first, second, and third shrinkage zones can be set in the analytical model based on the maximum temperature distribution. In this case, the zone where the maximum temperature is equal to or higher than T₁ can be the first shrinkage zone, the zone where the maximum temperature is lower than T₁ and equal to or higher than T₂ can be the second shrinkage zone, and a zone where the maximum temperature is lower than T₂ and equal to or higher than a third temperature T₃ can be the third shrinkage zone. Similarly, four or more shrinkage zones (for example, first to fourth shrinkage zones, first to fifth shrinkage zones, first to sixth shrinkage zones, first to seventh shrinkage zones, first to eighth shrinkage zones, first to ninth shrinkage zones, first to tenth shrinkage zones) can be set based on the maximum temperature distribution. When the object is heated multiple times, each shrinkage zone can be set for each heating.

Next, the change in temperature ΔT of the first shrinkage zone is set and the change in temperature ΔT of the second shrinkage zone is set based on the maximum temperature distribution. The change in temperature ΔT of the first shrinkage zone is different from the change in temperature ΔT of the second shrinkage zone. In addition, when three or more shrinkage zones are set, the change in temperature ΔT can be set for each set shrinkage zone based on the maximum temperature distribution. The change in temperature ΔT of each shrinkage zone is different from the change in temperature ΔT of the other shrinkage zones. Furthermore, when the object is heated multiple times, the change in temperature ΔT of each shrinkage zone can be set based on the maximum temperature distribution for each heating.

The change in temperature ΔT can be, for example, a temperature difference between the maximum temperature in the shrinkage zone and room temperature. The maximum temperature in the shrinkage zone may be an average value of the maximum temperature in the shrinkage zone, a lower limit of the maximum temperature in the shrinkage zone, or a median of a temperature range of the maximum temperature in the shrinkage zone.

When the object is heated multiple times and the temperature of the object does not drop to room temperature between the heatings, the change in temperature ΔT can also be a temperature difference between the maximum temperature in the shrinkage zone and a preset temperature.

Next, the elastic analysis or the elastic-plastic analysis is performed by uniformly giving the shrinkage strain ε = αΔT (α: linear expansion coefficient) calculated from the set change in temperature ΔT to the corresponding shrinkage zone. For example, idealized explicit FEM can be used for this analysis. In addition, the shrinkage strain can be isotropically given to it in three axial directions.

For example, when the first shrinkage zone and the second shrinkage zone are set, the elastic analysis or the elastic-plastic analysis is performed by uniformly giving the first shrinkage strain to the first shrinkage zone and by uniformly giving the second shrinkage strain to the second shrinkage zone. This makes it possible to simulate a shrinkage during the cooling process of the object, and to calculate the deformation and the residual stress distribution caused by the shrinkage. When multiple shrinkage zones are set, the elastic analysis or the elastic-plastic analysis is performed by uniformly giving the corresponding shrinkage strain to each shrinkage zone.

In addition, when the object is heated multiple times (for example, multi-layer welding, multi-pass welding, metal additive manufacturing, etc.), the elastic analysis or the elastic-plastic analysis can be performed sequentially for each heating according to the heating order. In this case, the final deformation or the final residual stress distribution can be calculated by the final elastic analysis or the final elastic-plastic analysis. In the flowchart of FIG. 2, the first to n-th welding passes are formed, so that the elastic analysis or the elastic-plastic analysis can be performed sequentially for each welding pass.

### Prediction of Deformation and Residual Stress

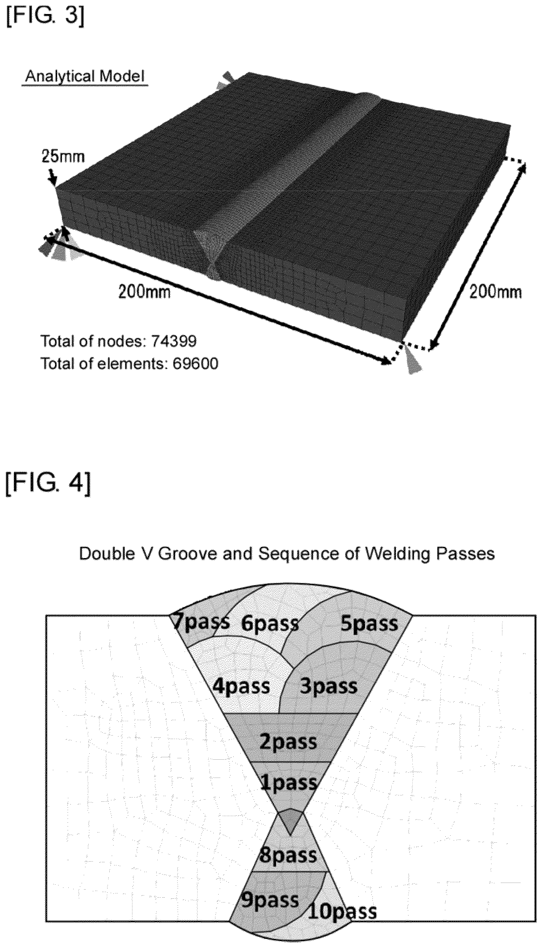

An analytical model (multi-layer butt welding model, length: 200 mm, width: 200 mm, thickness: 25 mm) as shown in FIG. 3 was created. In this analytical model, the joint surface of the base material was welded by forming 10 welding passes on the base material that has been subjected to edge preparation by arc welding. The material of the object to be analyzed is steel SM490A. FIG. 4 is a cross-sectional view of the welded portion of the analytical model shown in FIG. 3, showing a formation sequence of the 10 welding passes. In this analytical model, the first to seventh welding passes were formed on the groove portion from the top side of the analytical model, and then after gouging from the bottom side, the eighth to tenth welding passes were formed from the bottom side.

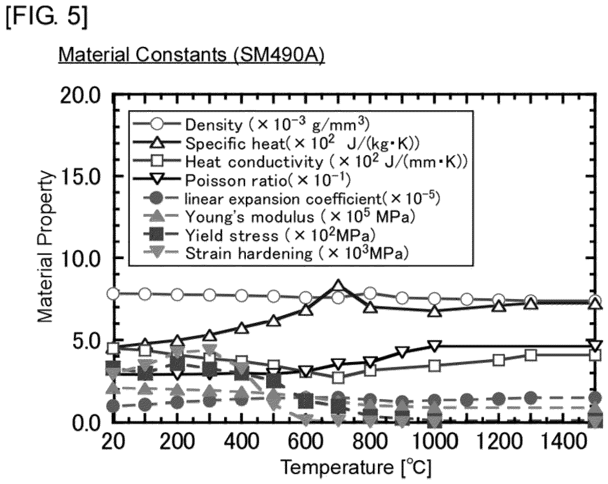

Next, a heat conduction analysis was performed using this analytical model to calculate a maximum temperature distribution when forming each welding pass (from when welding is performed until the welding pass returns to room temperature). Figure 5 is a graph showing material constants used in the analysis. Table 1 shows heat input conditions for each welding pass.

**[Table 1]**

| Pass No. | Electric current [A] | Voltage [V] | Welding speed [mm/sec] | Efficiency |
|---|---|---|---|---|
| 1 | 170 | 23 | 2.75 | 0.7 |
| 2~7 | 205 | 27 | 3.00 | |
| 8~9 | 190 | 27 | 2.00 | |
| 10 | 190 | 27 | 3.58 | |

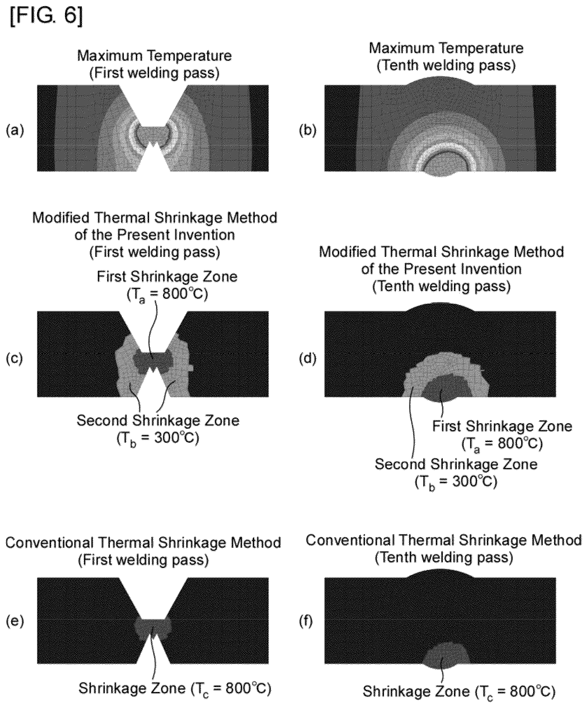

Figure 6(a) shows a maximum temperature distribution in the formation of the first welding pass, and Figure 6(b) shows a maximum temperature distribution in the formation of the tenth welding pass. In addition, a maximum temperature distribution in the formation of each of the second to ninth welding passes was also created (not shown). The maximum temperature in the part where the metal melted when forming the welding pass was 800°C or higher, and the maximum temperature decreased with increasing distance from this melted part.

Next, in the analysis using the modified thermal shrinkage method of the present invention, a first shrinkage zone and a second shrinkage zone were set in each analytical model after the formation of the first, second, third, fourth, fifth, sixth, seventh, eighth, ninth, or tenth welding pass from the calculated maximum temperature distributions. Specifically, a zone where the maximum temperature was 800°C or more in the maximum temperature distribution was set as the first shrinkage zone (maximum temperature Tₐ = 800°C), and a zone where the maximum temperature was 300°C or more and less than 800°C in the maximum temperature distribution was set as the second shrinkage zone (maximum temperature T_{b} = 300°C). A change in temperature ΔT (change in temperature from the maximum temperature to room temperature) of the first shrinkage zone was set to 800°C, and a change in temperature ΔT of the second shrinkage zone was set to 300°C. Figure 6(c) is a cross-sectional view of the analytical model showing the first and second shrinkage zones set in the analytical model after forming the first welding pass in the analysis using the modified thermal shrinkage method of the present invention, and Figure 6(d) is a cross-sectional view of the analytical model showing the first and second shrinkage zones set in the analytical model after forming the tenth welding pass in the analysis using the modified thermal shrinkage method of the present invention.

Next, an elastic-plastic analysis was performed by giving a first shrinkage strain ε₁ = αΔT (α: linear expansion coefficient, ΔT: the change in temperature of the first shrinkage zone) to the first shrinkage zone of the analytical model after the first welding pass was formed, and by giving a second shrinkage strain ε₂ = αΔT (α: linear expansion coefficient, ΔT: the change in temperature of the second shrinkage zone) to the second shrinkage zone of the analytical model after the first welding pass was formed to calculate a deformation and a residual stress. After that, an elastic-plastic analysis was performed by giving a first shrinkage strain ε₁ = αΔT (α: linear expansion coefficient, ΔT: the change in temperature of the first shrinkage zone) to the first shrinkage zone of the analytical model after the second welding pass was formed, and by giving the second shrinkage strain ε₂ = αΔT (α: linear expansion coefficient, ΔT: the change in temperature of the second shrinkage zone) to the second shrinkage zone of the analytical model after the second welding pass was formed to calculate a deformation and a residual stress. An elastic-plastic analysis like these was also performed sequentially on each of the analytical model after the third welding pass, the analytical model after the fifth welding pass, the analytical model after the sixth welding pass, the analytical model after the seventh welding pass, the analytical model after the eighth welding pass, the analytical model after the ninth welding pass, and the analytical model after the tenth welding pass to calculate a deformation (angular distortion and transverse shrinkage) and a residual stress.

The linear expansion coefficient a is the value shown in the graph in Figure 5. An idealized explicit FEM was used for the elastic-plastic analyses.

For comparison, an analysis was performed using the conventional thermal shrinkage method. In this analysis, only one shrinkage zone was set in each analytical model after the first, second, third, fourth, fifth, sixth, seventh, eighth, ninth, or tenth welding pass was formed. Specifically, a zone where the maximum temperature was 800°C or more in the maximum temperature distribution was set as a shrinkage zone (maximum temperature Tc = 800°C). In addition, a change in temperature ΔT of the shrinkage zone (change in temperature from the maximum temperature to room temperature) was set to 800°C. Figure 6(e) is a cross-sectional view of the analytical model in which the shrinkage zone is set in the analytical model after the first welding pass is formed in the analysis using the conventional thermal shrinkage method, and Figure 6(f) is a cross-sectional view of the analytical model in which the shrinkage zone is set in the analytical model after the tenth welding pass is formed in the analysis using the conventional thermal shrinkage method. In the analysis using the conventional thermal shrinkage method, an elastic-plastic analysis was performed by sequentially giving a shrinkage strain to the zone of each of the analytical models after the first to tenth welding passes were formed to calculate a deformation (angular deformation and transverse shrinkage) and a residual stress. In addition, idealized explicit FEM was used for the elastic-plastic analysis.

In addition, a deformation (angular deformation and transverse shrinkage) and a residual stress were calculated using a thermal elastic-plastic analysis.

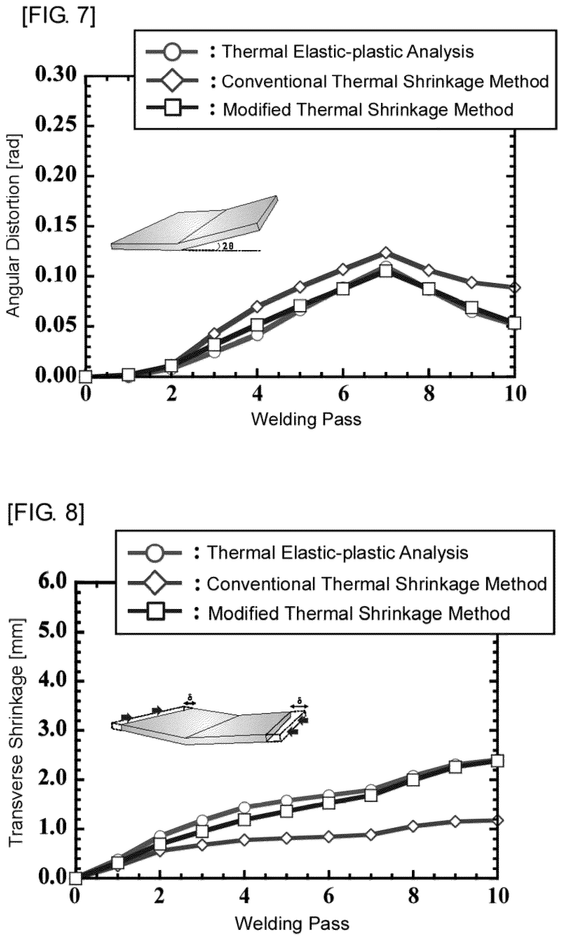

Figure 7 is a graph showing angular deformation histories when welding passes are sequentially formed, showing the angular deformation history calculated using the thermal elastic-plastic analysis, the angular deformation history calculated by the analysis using the conventional thermal shrinkage method, and the angular deformation history calculated by the analysis using the modified thermal shrinkage method of the present invention. As shown in the graph in Figure 7, it is found that the angular deformation calculated by the analysis using the modified thermal shrinkage method of the present invention is consistent with the angular deformation calculated by the thermal elastic-plastic analysis. In addition, the angular deformation calculated by the analysis using the conventional thermal shrinkage method is larger than the angular deformation calculated by the thermal elastic-plastic analysis.

Figure 8 is a graph showing transverse shrinkage histories when welding passes are formed sequentially, showing the transverse shrinkage history calculated using the thermal elastic-plastic analysis, the transverse shrinkage history calculated by the analysis using the conventional thermal shrinkage method, and the transverse shrinkage history calculated by the analysis using the modified thermal shrinkage method of the present invention. As shown in the graph in Figure 8, it is found that the transverse shrinkage calculated by the analysis using the modified thermal shrinkage method of the present invention is consistent with the transverse shrinkage calculated by the thermal elastic-plastic analysis. In addition, the transverse shrinkage calculated by the analysis using the conventional thermal shrinkage method is smaller than the transverse shrinkage calculated by the thermal elastic-plastic analysis.

Therefore, it is found that by performing the analysis using the modified thermal shrinkage method of the present invention, it is possible to calculate deformation that is consistent with the analysis results of the thermal elastic-plastic analysis in a short period of time.

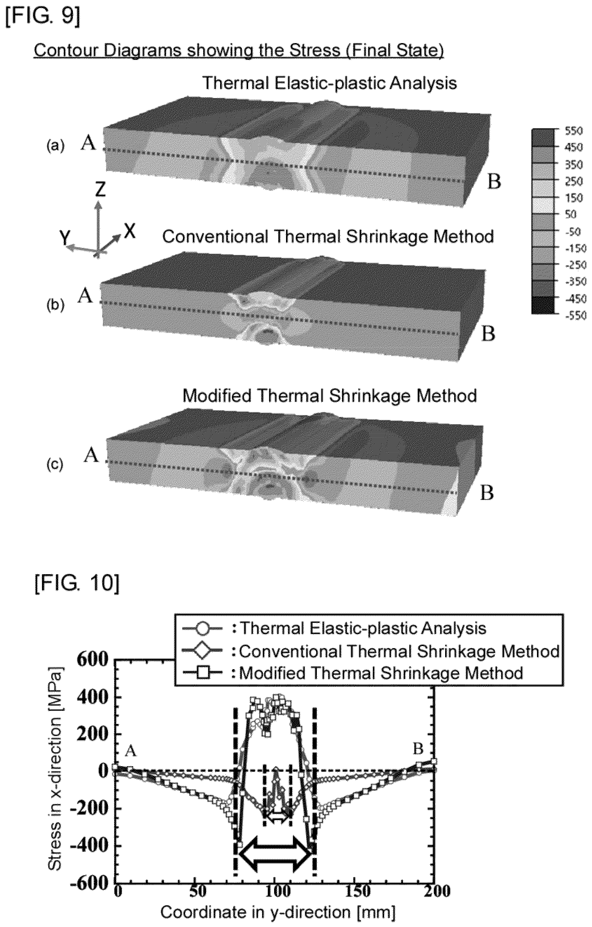

Figures 9(a) to (c) are contour diagrams showing residual stress distributions calculated for the analytical model after the tenth welding pass is formed, where FIG. 9(a) shows the residual stress distribution calculated using the thermal elastic-plastic analysis, FIG. 9(b) shows the residual stress distribution calculated by the analysis using the conventional thermal shrinkage method, and FIG. 9(c) shows the residual stress distribution calculated by the analysis using the modified thermal shrinkage method of the present invention.

Also, FIG. 10 is a graph showing the residual stress in the X direction at the dotted line A-B shown in FIGS. 9(a) to (c), and shows the residual stress distributions calculated by the thermal elastic-plastic analysis, the analysis using the conventional thermal shrinkage method, and the analysis using the modified thermal shrinkage method of the present invention.

The residual stress distribution calculated by the analysis using the modified thermal shrinkage method of the present invention shows the same tendency as the residual stress distribution calculated by the thermal elastic-plastic analysis. On the other hand, the residual stress distribution calculated by the analysis using the conventional thermal shrinkage method has a large deviation from the residual stress distribution calculated by the thermal elastic-plastic analysis.

Therefore, it is found that by performing the analysis using the modified thermal shrinkage method of the present invention, it is possible to calculate a residual stress distribution that shows the same tendency as the analysis results of the thermal elastic-plastic analysis in a short period of time.

## Claims

1. A method for predicting a deformation or a residual stress caused by returning temperature of an object subjected to heating to room temperature, comprising:
a condition setting step of setting a first shrinkage zone and a second shrinkage zone in an analytical model of the object, and setting a first change in temperature of the first shrinkage zone and a second change in temperature of the second shrinkage zone; and
an analysis step of performing an elastic analysis or an elastic-plastic analysis by giving a first shrinkage strain calculated from the first change in temperature to the first shrinkage zone and by giving a second shrinkage strain calculated from the second change in temperature to the second shrinkage zone.

2. The method according to claim 1, further comprising a step of calculating a maximum temperature distribution of the object, wherein the condition setting step is a step of setting the first shrinkage zone, the second shrinkage zone, the first change in temperature, and the second change in temperature based on the maximum temperature distribution.

3. The method according to claim 2, wherein the condition setting step is a step of setting a zone in which the maximum temperature is equal to or higher than a first temperature T₁ as the first shrinkage zone, and setting a zone in which the maximum temperature is lower than the first temperature T₁ and higher than a second temperature T₂ as the second shrinkage zone.

4. The method according to claim 1, wherein
the condition setting step includes a step of setting a third shrinkage zone in the analytical model, and setting a third change in temperature of the third shrinkage zone, and
the analysis step includes a step of performing the elastic analysis or the elastic-plastic analysis by giving a third shrinkage strain calculated from the third change in temperature to the third shrinkage zone.

5. The method according to claim 4, wherein
the condition setting step is a step of setting a plurality of shrinkage zones in the analytical model, and setting a change in temperature of each shrinkage zone,
the analysis step is a step of performing the elastic analysis or the elastic-plastic analysis by giving a shrinkage strain calculated from each change in temperature of the corresponding shrinkage zone, and
the plurality of shrinkage zones includes the first, second, and third shrinkage zones.

6. The method according to claim 5, further comprising a step of calculating a maximum temperature distribution of the object, wherein the condition setting step is a step of setting the plurality of shrinkage zones and the change in temperature of each shrinkage zone based on the maximum temperature distribution.

7. The method according to claim 1, being a method for predicting the deformation or the residual stress caused by returning temperature of the object subjected to multiple heating to room temperature, wherein
the condition setting step is performed for each heating, and
the analysis step is performed sequentially for each heating according to the heating order.

8. The method according to claim 1, wherein the elastic analysis or the elastic-plastic analysis in the analysis step is performed with idealized explicit FEM.

9. A program adapted to cause a computer to execute the method according to any one of claims 1 to 8.
